# EUROPEAN PATENT SPECIFICATION

(11) **EP 3 395 800 B1**
(45) Date of publication and mention of the grant of the patent: **03.11.2021**
(21) Application number: 17168220.6
(22) Date of filing: 26.04.2017
(51) Int. Cl.: C07D 233/60, C08G 65/333, C09D 163/02, C09D 175/04

(54) **HETEROCYCLIC QUATERNARY NITROGEN COMPOUNDS COMPRISING A POLYMERIC SUBSTITUENT AND THEIR USE AS A PHOTOLATENT CATALYST IN CURABLE COMPOSITIONS**
EINEM POLYMEREN SUBSTITUENTEN BEINHALTENDE HETEROZYCLISCHE QUATERNÄRE STICKSTOFFVERBINDUNGEN UND IHRE VERWENDUNG ALS PHOTOLATENTER KATALYSATOR IN HÄRTBAREN ZUSAMMENSETZUNGEN
COMPOSÉS AZOTÉS QUATERNAIRES HÉTEROCYCLIQUES COMPRENANT UN SUBSTITUANT POLYMÉRIQUE ET LEUR UTILISATION EN TANT QUE CATALYSEUR PHOTOLATENT DANS DES COMPOSITIONS DURCISSABLES

(43) Date of publication of application: 31.10.2018
(73) Proprietor: Henkel AG & Co. KGaA, 40589 Düsseldorf (DE)
(72) Inventor: ZHAO, Ligang, 40593 Düsseldorf (DE); DEL RIO NIETO, Enrique, 37764 Valero, Salamanca (ES); VERA SAZ, Francisco, 50018 Zaragoza (ES); BERGES SERRANO, Cristina, 500100 Zaragoza (ES)

(56) References cited:
- WO-A1-98/38195
- WO-A1-2009/156277
- WO-A1-2013/170857
- WO-A1-2016/089487
- US-A- 4 212 764
- Zhe Jia ET AL: "Optimizing the electrochemical performance of imidazolium-based polymeric ionic liquids by varying tethering groups", JOURNAL OF POLYMER SCIENCE, PART A: POLYMER CHEMISTRY, vol. 53, no. 11, 1 June 2015 (2015-06-01), pages 1339-1350, XP055611277, US ISSN: 0887-624X, DOI: 10.1002/pola.27567
- Qiang Zhao ET AL: "Supporting Information Poly(ionic liquid) Complex with Spontaneous Micro- /Mesoporosity: Template-Free Synthesis and Application as Catalyst Support", , 10 July 2012 (2012-07-10), XP055611283, Retrieved from the Internet: URL:https://pubs.acs.org/doi/suppl/10.1021 /ja303552p/suppl_file/ja303552p_si_001.pdf [retrieved on 2019-08-06]
- Javier L. Urraca ET AL: "Polymeric Complements to the Alzheimer's Disease Biomarker &bgr;-Amyloid Isoforms A&bgr;1-40 and A&bgr;1-42 for Blood Serum Analysis under Denaturing Conditions", Journal of the American Chemical Society, vol. 133, no. 24, 22 June 2011 (2011-06-22), pages 9220-9223, XP055009756, ISSN: 0002-7863, DOI: 10.1021/ja202908z
- ZHAO AU ET AL: "Electropolymerization of viologen derivatives and their electrochemical properties on electrodes", QINGDAO DAXUE XUEBAO, GONGCHENG JISHUBAN, QINGDAO DAXUE, CN , vol. 11, no. 3 1 January 1996 (1996-01-01), pages 1-12, XP009518968, ISSN: 1006-9798 Retrieved from the Internet: URL:file:///C:/Users/BW50982/Downloads/199 6_Wenyuan_SynthesisofElectropolymerizablea ndElectroactivebipyridiniumsalts.pdf
- Yasuhiro Amemiya ET AL: "The cyclo-copolymerization of diallyl compounds and sulfur dioxide, 5. Copolymerization of some 4-substituted 1,6-heptadiene derivatives with sulfur dioxide", Makromol. Chem and Physics, vol. 178, no. 2, 1 February 1977 (1977-02-01), pages 289-315, XP055669742, ISSN: 0025-116X, DOI: 10.1002/macp.1977.021780202
- BAPTISTE THIERRY ET AL: "Cinchona alkaloid-based polymer-bound phase-transfer catalysts: Efficient enantioselective alkylation of benzophenone imine of glycine esters", MOLECULAR DIVERSITY, KLUWER ACADEMIC PUBLISHERS, DO, vol. 9, no. 4, 1 November 2005 (2005-11-01), pages 277-290, XP019258622, ISSN: 1573-501X

## Description

### FIELD OF THE INVENTION

This application is directed to a quaternary nitrogen compound which may be used as a latent catalyst for curable compositions. In particular, the present application is directed to a metal-free, latent catalyst which comprises at least one quaternary nitrogen compound, which catalyst is activatable under ultraviolet irradiation and which finds particular utility in curable polyurethane or epoxy-resin compositions.

### BACKGROUND TO THE INVENTION

In recent decades, polymers have found utility in ever more sophisticated applications, replacing or complementing more traditional materials. This growing demand has driven the rapid development in polymer processing technologies which are to be applied in *inter alia:* the manufacture of composite materials; coating, adhesive and sealant technologies; reaction-injection-molding; and, the like. All these technologies depend on control over either the polymerization reaction or a crosslinking or curing reaction and one of the challenges for polymer chemists has been the design of catalysts for such reactions that can be *"switched on"* from an inactive state by application of an external stimulus, such as light, heat, oxygen or moisture.

The primary advantage of such latent catalytic systems which are activatable on demand is that they can facilitate control of the onset of a reaction to the exact place where and time when it is desired. Conversely, the use of conventional catalysts to accelerate a given polymerization or cross-linking reaction is limited by the need to balance fast and efficient curing with sufficient pot-life for handling and, if necessary, the application of the reactive system.

A supplementary advantage of latent catalytic systems is that they may provide enhanced production safety and simplify the technological setup as the problematic *in situ* addition and mixing of (highly) reactive chemicals can be avoided. Further, latent catalysts can be stored together with the monomers, curative compounds or cross-linking agents without premature reaction occurring; in turn, this can enable the development of single-component formulations that are ready to polymerize or cure, simply by application of the appropriate external stimulus.

Whilst these advantages are of course desirable, their realization is often difficult in practice. For instance, in both laboratory and industrial settings, physicochemical processing conditions tend to be highly variable and a given latent catalyst may not function as effectively across the whole range of conditions found.

Furthermore, there are conflicting requirements driving the selection of the most appropriate latent catalyst: a formulator will desire a fast and quantitative catalyst activation, which means that the transition from perfect latency to high activity has to be swift, but that activatable catalyst must conversely be stable enough to remain inert under storage conditions, often in a chemical environment that contains fillers, stabilizers, solvents and other additives.

The present invention is primarily concerned with coating, sealant and adhesive compositions which are to be cured using, as a catalyst, a base generated by photo-irradiation and, in particular UV irradiation. Establishing the most appropriate latent catalyst in this specific context can present supplementary difficulties to those outlined above. Suitable latent catalysts must be sensitive enough to UV light in their specific physicochemical environment; however, conventional photo-base generators usually present low solubility in some resins, leading to a lack of homogeneity in their distribution and thus low efficiency of the curing reaction. Conversely, where the latent catalysts generate low molecular weight species upon exposure to the stimulus of UV-light, such species can show significant mobility in the formulation; the migration of low molecular weight species can be problematic for certain applications, especially those in the food or health fields.

It is conceivable that the efficiency of the base-release reaction upon UV irradiation - and thereby the subsequent base-catalyzed reaction - can be enhanced by designing latent catalysts which exhibit better solubility in the photo-curable resin. A strategy mentioned by certain authors to attain a high degree of solubility is to introduce long alkyl chains as substituents in the tertiary amines or in the quaternary ammonium salts. For example, US Patent No. 6,087,070 (Turner et al.) describes organic compounds which can act as photoinitators for base catalyzable reactions, which have a molecular weight of less than 1000 and which in Formula (II) may optionally be modified with a plurality of C1-C18 alkyl groups (R², R³, R⁵, R¹⁷ and R¹⁸). Further, WO 98/38195 (Ciba Geigy AG) describes α-ammonium ketones, iminium ketones or amidinium ketones in the form of their tetraaryl or triarylalkylborate salts, which ketones can be photochemically converted into amines, imines or amidines and have alkyl groups as substituents of 18 carbons or less. However, it is submitted that alkyl chains having a length of 18 carbons or less may not have a sufficient length to adequately influence the solubility of the final compound. In addition, WO 2009156277 A1 discloses salt modified copolymers wherein one of the monomer units contains a quaternized N-atom structure with a polar substituent and the use of said copolymers as dispersant especially for color filters. The polar substituent is selected from selected from polyether, polyamine, nitrile, amide, imine, imide, ester, ketone, nitrile, aldehyde, diketone, ketoester, ketoamide, carbonate, carbamate, carbamide, sulfoxide, sulfone, carboxylic acid, sulfonic acid, phosphoric acid groups; or carboxylic acid anion, sulfonic acid anion or phosphoric acid anion groups which is formed as betaine structure. Moreover, WO 2013/170857 A1 describes polymeric photoinitiators where the photoinitiator moieties are incorporated as pendant groups on the polymeric backbone, as well as photoinitiator monomers being intermediates in the preparation of such polymeric photoinitiators. In the photoinitiator monomers and polymeric photoinitiators, a photoinitiator moiety and a quaternary ammonium group are incorporated into the photoinitiator structure, such that the photoinitiator moieties are present as pendant groups on the polymeric backbone when used in polymers.

It is postulated that migration issues can be controlled by incorporating a polymerizable group into the photo-base generator structure. Suyama et al. in Reactive & Function Polymers, 2013, 73, Pages 518-523 reports a way to reduce the migration of small molecules such as ketones by synthesizing a monomeric photo-base generator (4-vinylacetophenone O-phenylacetyloxime) which is copolymerized with glycidyl methacrylate. This strategy can, however, minimize the efficiency of the catalyst due to its reduced mobility as it is introduced into the polymeric chain.

In certain types of polymerizations involving photoinitiators - radical polymerizations - the risks associated with photoinitiator migration can be reduced by introducing lower concentrations of such photoinitators and compensating for this by including monomers of enhanced reactivity. WO 2015/148094 (Sun Chemical Corporation et al.), for example, discloses a curable composition by radical polymerization containing highly alkoxylated monomers.

### STATEMENT OF THE INVENTION

In accordance with a first aspect of the present invention, there is provided a quaternary nitrogen compound comprising a nitrogen heterocycle wherein: at least one polymeric substituent is bound to a ring atom of said heterocycle; and, an aromatic photoremovable group (PRG) is directly bound to a quaternary nitrogen ring atom of said heterocycle, the release of said aromatic photoremovable group under UV irradiation yielding a tertiary amine, in which said aromatic photoremovable group (PRG) is represented by the formula:

-CR¹R²-Ar (PRG)

wherein: R¹ and R² are independently of one another hydrogen or C1-C6 alkyl;
Ar represents an aryl group having from 9 to 18 ring carbon atoms, which aryl group may be unsubstituted or may be substituted by one of more C1-C6 alkyl group, C2-C4 alkenyl group, CN, OR³, SR³, CH₂OR³, C(O)R³, C(O)OR³ or halogen; and,
where present, each R³ is independently selected from the group consisting of hydrogen, C1-C6-alkyl and phenyl,
in which the at least one polymeric substituent is selected from polyethers, polyesters, polycarbonates, and copolymers thereof, and
the nitrogen heterocycle is an unsaturated monocyclic or bicyclic nitrogen heterocycle having at least two ring nitrogen atoms.
In an important embodiment of the compound, it comprises a stoichiometric amount of a counter ion of anionic charge selected from halides and non-coordinating anions comprising an element selected from boron, phosphorous or silicon.

In a number of important embodiments, the quaternary nitrogen compound is denoted by the general formula:

Y-(L-β-PRG)ᵣ

wherein: Y is an r-valent polymeric radical selected from the group consisting of polyethers, polyesters, polycarbonates, and copolymers thereof;
L is a covalent bond or an organic linking group;
β represents a nitrogen heterocycle having a quaternary nitrogen ring atom with which is associated a charge balancing anion;
PRG is an aromatic photoremovable group bound to a quaternary nitrogen ring atom of said heterocycle, in which said aromatic photoremovable group (PRG) is represented by the formula:

-CR¹R²-Ar (PRG)

wherein: R¹ and R² are independently of one another hydrogen or C1-C6 alkyl;
Ar represents an aryl group having from 9 to 18 ring carbon atoms, which aryl group may be unsubstituted or may be substituted by one of more C1-C6 alkyl group, C2-C4 alkenyl group, CN, OR³, SR³, CH₂OR³, C(O)R³, C(O)OR³ or halogen; and,
where present, each R³ is independently selected from the group consisting of hydrogen, C1-C6-alkyl and phenyl; and,
r is an integer of at least 1, preferably an integer of from 1 to 4.

Desirably, the r-valent polymeric radical Y is a polyether selected from group consisting of polyalkylene oxides and copolymers thereof. And in an advantageous embodiment, said r-valent polymeric radical Y is either a linear homopolymer of ethylene oxide or propylene oxide or a linear copolymer of ethylene oxide, propylene oxide and, optionally, butylene oxide.

Generally the at least one polymeric substituent or, where applicable, the r-valent polymeric radical Y of the quaternary ammonium compound is characterized by having a weight average molecular weight (Mw) of from 100 to 100,000, preferably from 400 to 7500 g/mol. The provision of substituents of this molecular weight significantly impacts the solubility of the quaternary nitrogen compound in curable compositions or other resinous systems. Further, the tertiary amine released upon irradiation of the quaternary nitrogen compound with UV-light, will still contain the polymeric substituents: the immediate mobility of the tertiary amine in the curable composition or resinous system may be improved as compared to the quaternary nitrogen compound but the retention of the polymeric substituents will inhibit its significant migration.

The present invention also recognizes that the molecular weight of the at least one polymeric substituent is a result effective variable which may be tailored, dependent on the curable compositions or other resinous systems in which the quaternary nitrogen compound may be included, to optimize the homogeneity of the compound's distribution therein.

In accordance with a second aspect of the present invention, there is provided the use of the compound as defined herein before and in the appended claims as a latent catalyst in a curable composition, the curing of which may be catalyzed or co-catalyzed by tertiary amines. In said use, the active form of the catalyst may be released when the quaternary nitrogen compound is irradiated with ultraviolet light having: a wavelength of from 150 to 600 nm, preferably from 200 to 450 nm; and / or, an energy of from 5 to 500 mJ/cm², preferably from 50 to 400 mJ/cm².

In accordance with a third aspect of the present invention, there is provided a polyurethane coating, adhesive or sealant composition comprising: a) a polyisocyanate; b) a polyol; and, c) a latent catalyst comprising at least one quaternary nitrogen compound as defined herein before and in the appended claims.

In accordance with a fourth aspect of the present invention, there is provided a curable epoxy-resin composition for a coating, adhesive or sealant composition or as a matrix for composite materials said epoxy resin composition comprising: a) an epoxy resin; b) a latent catalyst comprising at least one quaternary nitrogen compound as defined herein before and in the appended claims; and, optionally c) a curative for said epoxy resin.

The quaternary nitrogen compounds derived in accordance with the present invention exhibit a low activity during the processing of such curable compositions comprising them: such formulations thus exhibit sufficiently long pot-lives to enable their facile handling and application. Further, it has been found that the active catalyst(s) released after the quaternary nitrogen compounds are irradiated with UV-irradiation are sufficiently active to provide for a high curing rate. The catalysts are also not detrimental to the mechanical properties of the cured formulation.

### DEFINITIONS

As used herein, the singular forms "*a*", *"an"* and *"the"* include plural referents unless the context clearly dictates otherwise.

The terms *"comprising", "comprises"* and *"comprised* of" as used herein are synonymous with *"including", "includes", "containing"* or *"contains",* and are inclusive or open-ended and do not exclude additional, non-recited members, elements or method steps.

When amounts, concentrations, dimensions and other parameters are expressed in the form of a range, a preferable range, an upper limit value, a lower limit value or preferable upper and limit values, it should be understood that any ranges obtainable by combining any upper limit or preferable value with any lower limit or preferable value are also specifically disclosed, irrespective of whether the obtained ranges are clearly mentioned in the context.

The terms *"preferred", "preferably", "desirably", "in particular"* and *"particularly"* are used frequently herein to refer to embodiments of the disclosure that may afford particular benefits, under certain circumstances. However, the recitation of one or more preferable or preferred embodiments does not imply that other embodiments are not useful and is not intended to exclude those other embodiments from the scope of the disclosure.

The molecular weights given in the present text refer to weight average molecular weights (Mw), unless otherwise stipulated. All molecular weight data refer to values obtained by gel permeation chromatography (GPC), unless otherwise stipulated.

As used herein a *"quaternary nitrogen compound"* is a nitrogen molecular entity that is electronically neutral but which contains a quaternary nitrogen (https://www.ebi.ac.uk/chebi).

As used herein, the term "*co-catalyst*" refers to one or more catalysts that can be used in combination with a primary catalyst of the disclosed subject matter.

As used herein, *"aliphatic group"* means a saturated or unsaturated linear (i.e., straight-chain), branched, cyclic (including bicyclic) organic group: the term *"aliphatic group"* thus encompasses *"alicyclic group",* the latter being a cyclic hydrocarbon group having properties resembling those of an aliphatic group.

As used herein, "*C*₁-*C*₆ *alkyl*" group refers to a monovalent group that contains 1 to 6 carbons atoms, that is a radical of an alkane and includes straight-chain and branched organic groups. Examples of alkyl groups include, but are not limited to: methyl; ethyl; propyl; isopropyl; n-butyl; isobutyl; sec-butyl; tert-butyl; n-pentyl; and, n-hexyl. In the present invention, such alkyl groups may be unsubstituted or may be substituted with one or more substituents such as halo, nitro, cyano, amido, amino, sulfonyl, sulfinyl, sulfanyl, sulfoxy, urea, thiourea, sulfamoyl, sulfamide and hydroxy. The halogenated derivatives of the exemplary hydrocarbon radicals listed above might, in particular, be mentioned as examples of suitable substituted alkyl groups. In general, however, a preference for unsubstituted alkyl groups containing from 1-6 carbon atoms (C₁-C₆ alkyl) - for example unsubstituted alkyl groups containing from 1 to 4 carbon atoms (C₁-C₄ alkyl) or 1 or 2 carbon atoms (C₁-C₂ alkyl) - should be noted.

The term *"alkylene group"* refers to a divalent group that is a radical of an alkane and includes linear and branched organic groups, which groups may be substituted or substituted. In general, a preference for unsubstituted alkylene groups containing from 2-6 carbon atoms (C₂-C₆ alkylene) - for example unsubstituted alkylene groups containing from 2 to 4 carbon atoms (C₂-C₄ alkylene) - should be noted.

As used herein, "*C*₂-*C*₄ *alkenyl*" group refers to an aliphatic carbon group that contains 2 to 4 carbon atoms and at least one double bond. Like the aforementioned alkyl group, an alkenyl group can be straight or branched, and may optionally be substituted. Examples of C₂-C₄ alkenyl groups include, but are not limited to: allyl; isoprenyl; and, 2-butenyl.

As used herein, the term "*aryl*" refers to an aromatic ring wherein each of the atoms forming the ring is a carbon atom. Aryl rings can be formed by five, six, seven, eight, nine, or more than nine carbon atoms. Aryl groups can be optionally substituted. Examples of aryl groups include, but are not limited to phenyl, naphthalenyl, phenanthrenyl, anthracenyl, fluorenyl, and indenyl. Depending on the structure, an aryl group can be a monoradical or a diradical (i.e., an arylene group).

By the term *"arylaliphatic"* is meant a hydrocarbon moiety, in which one or more aromatic moieties are substituted with one or more aliphatic groups. Thus the term *"arylaliphatic"* also includes hydrocarbon moieties, in which two or more aryl groups are connected via one or more aliphatic chain or chains of any length, for instance a methylene group.

As used herein *"polyisocyanate"* means a compound comprising at least two -N=C=O functional groups, for example from 2 to 5 or from 2 to 4 -N=C=O functional groups. Suitable polyisocyanates include aliphatic, cycloaliphatic, aromatic and heterocyclic isocyanates, dimers and trimers thereof, and mixtures thereof.

Aliphatic and cycloaliphatic polyisocyanates can comprise from 6 to 100 carbon atoms linked in a straight chain or cyclized and having at least two isocyanate reactive groups. Examples of suitable aliphatic isocyanates include but are not limited to straight chain isocyanates such as ethylene diisocyanate, trimethylene diisocyanate, tetramethylene diisocyanate, 1,6-hexamethylene diisocyanate (HDI), octamethylene diisocyanate, nonamethylene diisocyanate, decamethylene diisocyanate, 1,6,11-undecanetriisocyanate, 1,3,6- hexamethylene triisocyanate, bis(isocyanatoethyl)-carbonate, and bis (isocyanatoethyl) ether. Exemplary cycloaliphatic polyisocyanates include, but are not limited to, dicyclohexylmethane 4,4'-diisocyanate (H₁₂MDI), 1-isocyanatomethyl-3-isocyanato-1,5,5-trimethylcyclohexane (isophorone diisocyanate, IPDI), cyclohexane 1,4-diisocyanate, hydrogenated xylylene diisocyanate (H₆XDI), 1-methyl-2,4-diisocyanato-cyclohexane, m- or p-tetramethylxylene diisocyanate (m-TMXDI, p-TMXDI) and dimer fatty acid diisocyanate.

The term *"aromatic polyisocyanate"* is used herein to describe organic isocyanates in which the isocyanate groups are directly attached to the ring(s) of a mono- or polynuclear aromatic hydrocarbon group. In turn the mono- or polynuclear aromatic hydrocarbon group means an essentially planar cyclic hydrocarbon moiety of conjugated double bonds, which may be a single ring or may include multiple condensed (fused) or covalently linked rings. The term aromatic also includes alkylaryl. Typically, the hydrocarbon (main) chain includes 5, 6, 7 or 8 main chain atoms in one cycle. Examples of such planar cyclic hydrocarbon moieties include, but are not limited to, cyclopentadienyl, phenyl, napthalenyl-, [10]annulenyl-(1,3,5,7,9-cyclodecapentaenyl-), [12]annulenyl-, [8]annulenyl-, phenalene (perinaphthene), 1,9-dihydropyrene, chrysene (1,2-benzophenanthrene). Examples of alkylaryl moieties are benzyl, phenethyl, 1-phenylpropyl, 2-phenylpropyl, 3-phenylpropyl, 1-naphthylpropyl, 2-naphthylpropyl, 3-naphthylpropyl and 3-naphthylbutyl.

Exemplary aromatic polyisocyanates include, but are not limited to: all isomers of toluene diisocyanate (TDI), either in the isomerically pure form or as a mixture of several isomers; naphthalene 1,5-diisocyanate; diphenylmethane 4,4'-diisocyanate (MDI); diphenylmethane 2,4'-diisocyanate and mixtures of diphenylmethane 4,4'-diisocyanate with the 2,4' isomer or mixtures thereof with oligomers of higher functionality (so-called crude MDI); xylylene diisocyanate (XDI); diphenyl-dimethylmethane 4,4'-diisocyanate; di- and tetraalkyl-diphenylmethane diisocyanates; dibenzyl 4,4'-diisocyanate; phenylene 1,3-diisocyanate; and, phenylene 1,4-diisocyanate.

It is noted that the term *"polyisocyanate"* is intended to encompass pre-polymers formed by the partial reaction of the aforementioned aliphatic, cycloaliphatic, aromatic and heterocyclic isocyanates with polyols to give isocyanate functional oligomers, which oligomers may be used alone or in combination with free isocyanate(s).

For completeness: a) a primary amine group is an atomic grouping of the type "-NH₂" (R-H); (b) a secondary amine group is an atomic grouping of the type "-NHR"; c) a tertiary amine group is an atomic grouping of the type "-NR₂"; and, d) an amino group is understood to mean an atomic grouping of the type "-NH₂".

The term *"heterocyclic"* as used in the context of the present invention refers to compounds having saturated and unsaturated mono- or polycyclic cyclic ring systems having 3-16 atoms wherein at least one ring atom is nitrogen. Optionally, the nitrogen atom(s) may be oxidized. The ring systems may be optionally substituted with one or more functional groups, as defined herein.

As used herein *"halide"* refers to fluoride, chloride, bromide or iodide.

A *"compatible non-coordinating anion"* ("NCA") is defined herein as an anion which either does not coordinate the cation or which is only weakly coordinated to the cation. Further, the phrase *"compatible non-coordinating anion"* specifically refers to an anion which, when functioning as a stabilizing anion in the latent catalyst system of the present invention, does not irreversibly transfer an anionic substituent or fragment thereof to the cation thereby forming a neutral byproduct or other neutral compound.

As used herein "*hydrocarbyl*" refers to a monovalent, linear, branched or cyclic group which contains only carbon and hydrogen atoms. Substituted hydrocarbyl radicals are radicals in which at least one hydrogen atom has been substituted with at least one functional group.

*"Halocarbyl radicals"* are radicals in which one or more hydrocarbyl hydrogen atoms have been substituted with at least one halogen (e.g. F, CI, Br, I) or halogen-containing group (e.g. CF₃). Substituted halocarbyl radicals are (halogen containing) radicals in which at least one halocarbyl hydrogen or halogen atom has been substituted with at least one functional group.

The term "*polyol*" as used herein shall include diols and higher functionality hydroxyl compounds. In the context of the compositions of the present invention, preferred polyols will have from 2 to 4 hydroxyl moieties. Further, the preferred polyols may include polyether polyols, polyester polyols, poly(alkylene carbonate)polyols, hydroxyl-containing polythioethers, polymer polyols, and mixtures thereof. The hydroxyl number of useful polyhydroxy compounds in the present disclosure will generally be from 20 to 850 mg KOH/g and preferably from 25 to 500 mg KOH/g.

The hydroxyl (OH) values given herein are measured according to Japan Industrial Standard (JIS) K-1557, 6.4.

As used herein, *"epoxy resin"* refers to a resin which contains 2 or more reactive, epoxy functional groups. Although not intending to be limited to specific epoxy resin structures, useful epoxy resins in accordance with the present disclosure include: bisphenol A epoxy resins; bisphenol F epoxy resins; phenol novolac epoxy resins; polycyclic epoxy resins, such as, dicyclopentadiene type epoxy resins; and, mixtures thereof. The epoxy resin may be a liquid, solid or mixture of both. Epoxy resins useful in the practice of the present invention are commercially available or can be made by techniques well known in the art.

The term *"nucleophilic substitution (S_{N}2)"* is used herein in accordance with its standard meaning in the art. Instructive references in this regard include but are not limited to: March et al. March's Advanced Organic Chemistry, 5th Edition (ISBN: 9780471720911); http://www.organic-chemistry.org/namedreactions/nucleophitic-substitution-sn1-sn2.shtm; and, http://www.chem.ucalgary.ca/courses/351/Carey5th/Ch08/ch8-4.html.

As used herein, *"composite material"* refers to materials made from two or more constituent materials with significantly different physical and / or chemical properties, that when combined, produce a material with characteristics different from the individual components. The individual components remain separate and distinct within the finished structure, for example, domains in a matrix. Herein, the term *"composite material"* is in particular intended to encompass a resinous matrix in which is disposed at least one solid particulate material having, for example, the physical shape of platelets, shavings, flakes, ribbons, rods, fibers, strips, spheroids, toroids, pellets and tablets.

Two-component compositions in the context of the present invention are understood to be compositions in which a first reactive component and second reactive component must be stored in separate vessels because of their reactivity. The two components are mixed only shortly before application and then react, under catalysis, with bond formation and thereby formation of a polymeric network. Other ingredients may, of course, be stored with the first component and/or the second component or separately from both said components.

Viscosities of the adhesive compositions and of pre-polymers as described herein are, unless otherwise stipulated, measured using the Brookfield Viscometer, Model RVT at standard conditions of 20°C and 50% Relative Humidity (RH). The viscometer is calibrated using silicone oils of known viscosities, which vary from 5,000 cps to 50,000 cps. A set of RV spindles that attach to the viscometer are used for the calibration. Measurements of the pre-polymer are done using the No. 6 spindle at a speed of 20 revolutions per minute for 1 minute until the viscometer equilibrates. The viscosity corresponding to the equilibrium reading is then calculated using the calibration.

As used herein, *"anhydrous"* means the relevant composition includes less than 0.25% by weight of water. For example the composition may contain less than 0.1% by weight of water or be completely free of water. The term *"essentially free of solvent"* should be interpreted analogously as meaning the relevant composition comprises less than 0.25% by weight of solvent.

### DETAILED DESCRIPTION OF THE INVENTION

The invention is defined by the appended claims and will now be described with reference to a number of more detailed embodiments

At its broadest, the present invention defines a quaternary nitrogen compound of claim 1 which may find utility as a latent catalyst which is activatable under ultraviolet irradiation. The quaternary nitrogen compound comprises a nitrogen heterocycle wherein an aromatic photoremovable group (PRG) is directly bound to a quaternary nitrogen ring atom of said heterocycle, the release of said aromatic photoremovable group under UV irradiation yielding a tertiary amine. To at least one ring atom of the nitrogen heterocyclic compound is bound a polymeric substituent, which polymeric substituent(s) should preferably be characterized by a molecular weight of from 100 to 100,000 and more preferably by a molecular weight of from 400 to 7500 g/mol.

The quaternary nitrogen compound comprises an unsaturated monocyclic or bicyclic nitrogen heterocycle having at least two ring nitrogen atoms. Monocyclic nitrogen heterocycles will preferably be characterized by comprising from 3 to 9 non-hydrogen atoms or more preferably from 5 to 9 non-hydrogen atoms: said 5 to 9 membered monocyclic rings should further be characterized by having 2 or 3 nitrogen heteroatoms therein. Bicyclic nitrogen heterocycles will have two fused rings and preferably be characterized by containing *in toto* from 7 to 12 non-hydrogen atoms and from 2 to 4 nitrogen heteroatoms. Preferably at least one and more preferably both of the fused rings will contain 5 or 6 non-hydrogen atoms. Nitrogen atoms may be disposed at the ring junctions of the bicyclic nitrogen heterocycles.

Within the quaternary nitrogen compound, the positively charged nitrogen atom is that nitrogen atom to which the aromatic photoremovable group is bound. The compound is electrically neutral on account of containing a stoichiometric amount of a counter ion of anionic charge and, preferably, that counter ion is selected from halides and non-coordinating anions comprising an element selected from boron, phosphorous or silicon. In selecting counter anions of this type, the quaternary nitrogen compound can be characterized as being metal-free.

In the present invention, the aromatic photoremovable group (PRG) is directly bonded to a quaternary nitrogen ring atom of the heterocycle and is represented by the formula:

-CR¹R²-Ar (PRG)

wherein: R¹ and R² are independently of one another hydrogen or C1-C6 alkyl;
Ar represents an aryl group having from 9 to 18 ring carbon atoms, which aryl group may be unsubstituted or may be substituted by one of more C1-C6 alkyl group, C2-C4 alkenyl group, CN, OR³, SR³, CH₂OR³, C(O)R³, C(O)OR³ or halogen; and,
where present, each R³ is independently selected from the group consisting of hydrogen, C1-C6-alkyl and phenyl, in which the at least one polymeric substituent is selected from polyethers, polyesters, polycarbonates, and copolymers thereof, and
the nitrogen heterocycle is an unsaturated monocyclic or bicyclic nitrogen heterocycle having at least two ring nitrogen atoms.

Preferably, Ar represents an aryl group selected from phenanthrenyl, anthracenyl, fluorenyl and indenyl.

As regards the aforementioned general formula (PRG), it is preferred that:
At least one of R¹ and R² is hydrogen; and
Ar represents an aryl group having from 9 to 18 ring carbon atoms, which aryl group may be unsubstituted or may be substituted by one of more C1-C6 alkyl group, C2-C4 alkenyl group, C(O)R³ or halogen.

As noted above, in a number of important embodiments, the quaternary ammonium compound may be denoted by the general formula:

Y-(L-β-PRG)ᵣ

wherein: Y is an r-valent polymeric radical selected from the group consisting of polyethers, polyesters, polycarbonates, and copolymers thereof;
L is a covalent bond or an organic linking group;
β represents a nitrogen heterocycle having a quaternary nitrogen ring atom with which is associated a charge balancing anion;
PRG is an aromatic photoremovable group bound to a quaternary nitrogen ring atom of said heterocycle, in which said aromatic photoremovable group (PRG) is represented by the formula:

-CR¹R²-Ar (PRG)

wherein: R¹ and R² are independently of one another hydrogen or C1-C6 alkyl;
Ar represents an aryl group having from 9 to 18 ring carbon atoms, which aryl group may be unsubstituted or may be substituted by one of more C1-C6 alkyl group, C2-C4 alkenyl group, CN, OR³, SR³, CH₂OR³, C(O)R³, C(O)OR³ or halogen; and,
where present, each R³ is independently selected from the group consisting of hydrogen, C1-C6-alkyl and phenyl; and,
r is an integer of at least 1, preferably an integer of from 1 to 4.

The integer r may be, for instance, from 1 to 3.

As the r-valent polymeric radical (Y), suitable polyesters include, but are not limited to, polyethylene terephthalate and polybutylene terephthalate. Suitable polycarbonates include, but are not limited to, those prepared by the reaction of diols, such as 1,3-propanediol, 1,4-butanediol, 1,6-hexanediol, diethylene glycol, triethylene glycol, tetraethylene glycol, or thiodiglycol, with phosgene or diaryl carbonates, such as diphenyl carbonate. Exemplary polycarbonates, including polyestercarbonates, are disclosed in: German Auslegeschriften 1,694,080, 1,915,908, and 2,221,751; German Offenlegungsschrift 2,605,024; US Patent No. 4,334,053; US Patent No. 6,566,428; and, Canadian Patent No. 1,173,998.

In an important embodiment, the r-valent polymeric radical (Y) is a polyether selected from group consisting of polyalkylene oxides and copolymers thereof. Exemplary but non-limiting polyalkylene oxides include linear homopolymers of ethylene oxide or propylene oxide and linear copolymers of ethylene oxide, propylene oxide and, optionally, butylene oxide.

In certain exemplary embodiments wherein the quaternary nitrogen compound the quaternary ammonium compound may be denoted by the aforementioned general formula Y-(L-β-PRG)ᵣ, the group -β-PRG may more particularly be represented by either: 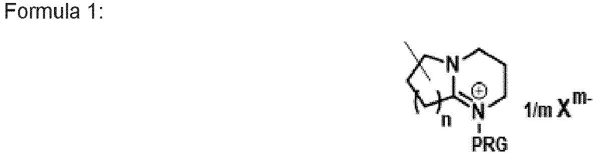 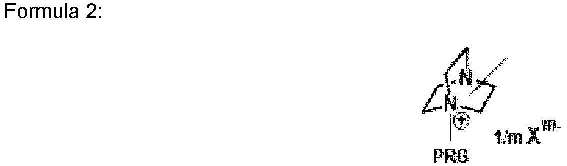 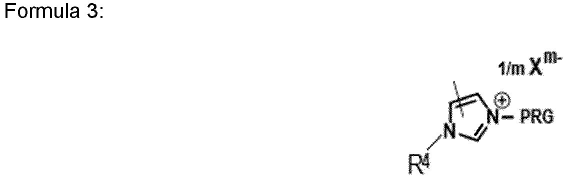 or, 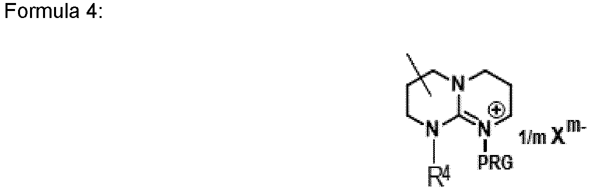
wherein: PRG is as defined above;
n is 1, 2 or 3;
R⁴ is hydrogen, C1-C6 alkyl, phenyl or a polymeric substituent which is represented by the general formula -L˙Y˙ in which L˙ is a covalent bond or an organic linking group and Y˙ is a polymeric radical selected from the group consisting of polyethers, polyesters, polycarbonates, and copolymers thereof; and,
X^{m-} is a counter ion of anionic charge m selected from halides and non-coordinating anions comprising an element selected from boron, phosphorous or silicon.

The polymeric radical (Y˙) may be the same as or different from the polyvalent radical (Y).

Said non-coordinating anions consist, typically, of a compound of said elements (B, P or Si) having a formal valence m' with more than m' radicals which independently may be: a hydride radical; a bridged or unbridged dialkylamido radical; an alkoxide and aryloxide radical; a hydrocarbyl or substituted hydrocarbyl radical; or, a halocarbyl or substitued halocarbyl radical. The charge of the anion equals the number of radicals minus the formal valence (m') of the (metalloid) element. The disclosure of US Patent No. 5,198,401 may be instructive on suitable non-coordinating anions of the elements B, P and Si.

For illustration, suitable boron-containing anions include: tetra(phenyl)borate; tetra(p-tolyl)borate; tetra(o-tolyl)borate; tetra(pentafluorphenyl)borate; tetra(o,p-dimethylphenyl)borate; tetra(m,m-dimethylphenyl)borate; and, tetra(p-trifluoromethylphenyl)borate.

### Synthesis of the Latent Catalysts

Without intention to limit the present invention, the quaternary nitrogen compounds are derivable from a multi-stage synthesis, of which Examples are provided herein under. A suitable synthetic method may be described as comprising the following stages:
i) forming an adduct (A) of a homo- or copolymer (Y) with an appropriate, defined nitrogen heterocyclic compound;
ii) reacting said adduct (A) with a halide-functional aralkylating agent to bind the aromatic photoremovable group (PRG) thereto; and,
iii) where applicable, swapping any halide anion present in the product of stage ii) with a non-coordinating anion (X').

### Stage i)

The first stage defined above will typically be constituted by the nucleophilic addition of a nucleophile to an electrophile. To effect this addition where the starting heterocyclic compound or the (co)polymer (Y) do not possess appropriate functional groups, one or both of these compounds may be functionalized with a group (L^{a}), the residue of which group will become incorporated in the adduct as linking group L.

Generally the adducts are prepared via either a nucleophilic substitution (S_{N}2) using an electrophile containing polymer or the Michael addition reaction of a molar excess of a functionalized polymer (Y- L^{a}) with the nitrogen heterocyclic compound, typically in the presence of a chlorinated hydrocarbon solvent and / or an aromatic solvent, such a toluene. Exemplary chlorinated hydrocarbon solvents include methylene chloride, ethylene dichloride, 1,1,1-trichloroethane and chloroform.

Depending on the functional groups present in the reacting monomers and also on inherent steric effects, it is possible for pendant electrophilic groups (L^{a}), and in particular (meth)acrylate groups, to be incorporated into polymers (Y) through copolymerization with ethylenically unsaturated monomers: suitable copolymerization methods include ionic polymerization, conventional radical polymerization, polycondensation and controlled radical polymerization (CRP). Alternately, the pendant electrophilic groups can be incorporated into the (co)polymer (Y) by making the polymer and then post-functionalizing it via subsequent reaction(s).

It is considered that polymerization processes and post-functionalization may be carried in facile manner by the skilled artisan. The following disclosures are also considered instructive in this regard: H. Mark, et al., Ed., Encyclopedia of Polymer Science and Engineering, 2nd Ed., Vol. 12, John Wiley & Sons, New York, 1988; H. Mark et al., Ed., Encyclopedia of Polymer Science and Engineering, 2nd Ed., Supplement Volume, John Wiley & Sons, New York, 1989.

For illustration, and without intention to limit the present invention, the reactant polymer of a Michael addition (Stage i)) may have the formula: 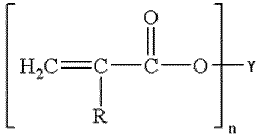
wherein: R is hydrogen or methyl;
n is ≧2; and,
Y is the homopolymer or copolymer described above, with the caveat that said (co)polymer
Y should not interfere with the Michael addition.

In this illustrative embodiment, it preferred that Y is selected from group consisting of polyalkylene oxides and copolymers thereof.

A base may optionally be used to catalyze the Michael addition reaction. Further, the Michael addition reaction can occur at room temperature but the rate of reaction can be increased at elevated temperatures, for example up to 100°C. The synthesis method is not so limited, however, and the reaction can be conducted at temperatures outside of these ranges. The reaction times can inevitably vary but a time frame of from 1 hour to 1 week may be considered typical: if necessary, the progress of the addition reaction can be followed by *inter alia* chromatography.

The crude product obtained from the addition reaction is desirably worked up prior to being employed in the further synthesis stages. As methods of separation and isolation of the Adduct (A) mention may be made of extraction, evaporation, re-precipitation, distillation and chromatography.

### Stage ii)

This stage of the illustrative synthesis method comprises the reaction of the Adduct (A) with a halide-containing aralkylating agent, which agent thereby binds the photoremovable group (PRG) to a nitrogen atom disposed in the heterocylic ring.

Substituted or unsubstituted chloroalkyl compounds and especially chloromethyl compounds of aromatic or partially hydrogenated aromatic compounds are particularly suitable as aralkylating agents in the present invention. As is known in the art, such chloromethyl derivatives of aromatic and partially hydrogenated aromatic hydrocarbons can be prepared by the introduction, under heating to 60-70°C, of hydrogen chloride gas into a mixture of the hydrocarbon, concentrated formaldehyde, concentrated aqueous hydrochloric acid and, optionally, zinc chloride. Exemplary chloromethyl derivatives include, but are not limited to: chloromethyl toluene; chloromethyl xylene; chloromethyl cumene; 2-chloromethyl cymene; 1-chloromethyl naphthalene; chloromethylcyclohexyl-benzene, obtainable, for example, by treatment of the addition product of benzene and cyclohexene with formaldehyde, aqueous hydrochloric acid and hydrogen chloride gas at 60-70°C.; chloromethyl anthracene; chloromethyloctahydroanthracene; and, chloromethyloctahydrophenanthrene.

Further known aralkylating agents useful in the present invention include: alkylbenzyl halides, such as octylbenzyl chloride; and, halogen ketones, such as chloroacetophenone and bromoacetophenone.

The reaction of the amines with the aralkylating agents is generally performed in the presence of an aprotic solvent and, optionally, acid-binding agents, such as sodium carbonate or calcium carbonate. The aprotic solvent should be stable to the action of the aralkylating agent and any such bases present and suitable solvents which may be mentioned in this regard include: simple linear ethers, such as diethyl ether and methyl-t-butyl ether; cyclic ethers, such as tetrahydrofuran and 1,4-dioxane; glyme ethers; amides, such as dimethylformamide; and, mixtures thereof.

Good results have been obtained where this aralkylation stage is performed under anhydrous conditions. If desired, exposure to atmospheric moisture may be avoided by providing the reaction vessel with an inert, dry gaseous blanket. Whilst dry nitrogen, helium and argon may be used as blanket gases, precaution should be used when common nitrogen gases are used as a blanket, because such nitrogen may not be dry enough on account of its susceptibility to moisture entrainment; the nitrogen may require an additional drying step before use herein.

The aralkylation reaction is normally performed at a temperature of from 20° to 100°C. The progress of the reaction can be monitored by *inter alia* chromatography: whilst the reactivity of the aralkylating agent is determinative of the duration of the reaction, a duration of from 2 to 48 hours will be standard.

After completion of the reaction, the crude reaction product may itself be used in the next, optional stage of the synthesis. However, the working up of the product is not precluded and an illustrative procedure would entail the evaporation of the solvent from the reaction mixture under sub-atmospheric pressure and the iterative (re-) precipitation of the product in a suitable solvent, such as diethyl ether. The product may, if desired, be further purified using methods known in the art such as extraction, evaporation, distillation and chromatography.

### Stage iii)

In the final synthesis stage, the halide counterion present after the aralkylation stage may be swapped with the aforementioned non-coordinating anions (hereinafter X').

The crude or separated and purified aralkylation product of stage ii) is contacted with a polar solvent. Such a solvent preferably comprises at least one compound selected from: alcohols, such as methanol or methoxyethanol; amide solvents, such as N,N-dimethylacetamide (DMA), N,N-dimethylformamide (DMF) and N-methylpyrrolidone (NMP); dimethylsulfoxide (DMSO); halocarbons, such as dichloromethane (DCM) and chloroform; esters, such as ethyl acetate (AcOEt) and isopropyl acetate (AcOiPr); ethers, such as diethyl ether, diisopropyl ether, dioxane, tetrahydrofuran (THF) and MTBE; pyridine; and, acetonitrile. Good results have been obtained where the solvent is selected from: methanol; methoxyethanol; N,N-dimethylacetamide (DMA); N-methylpyrrolidone (NMP); N,N-dimethylformamide (DMF); and, mixtures thereof.

The solution thus obtained is mixed with a salt (CatX'), wherein Cat denotes a cation selected from the group consisting of: protons (H⁺); alkali earth metal cations; and, alkaline earth metal cations. Cat is preferably an alkali metal cation and more particularly Na⁺ or K⁺.

In this synthesis stage, at least 1 mole, for example from 1 to 5 moles, of the salt (CatX') ought to be used per mole of the aralkylation product. It will be evident to the skilled partisan that smaller amounts of the salt (CatX') may be used but this may lead to only partial reaction of the aralkylation product. In any event, the desired amount of salt (CatX') may be introduced in solution, whereby the salt is preferably dissolved in the minimum amount of the solvent employed for the aralkylation product.

The solution obtained after adding the salt (CatX') is stirred until the reaction is complete, either at room temperature or at a slightly elevated temperature up to and including 80°C. The progress of the reaction can be monitored by chromatography, for example, but it is noted that a reaction duration of from 1 to 24 hours will be standard.

The crude product obtained may then be worked up, for which an illustrative procedure would entail the evaporation of the solvent from the reaction mixture under sub-atmospheric pressure, the dissolution of the obtained mixture in a suitable solvent, such as tetrahydrofuran, followed by filtration of the obtained solution to remove the halide salts; the filtrate may then be collected and the solvent evaporated there-from under reduced pressure. The so-obtained product may, if desired, be further purified using methods known in the art such as extraction, evaporation, re-crystallization, distillation and chromatography.

### Methods and Applications

An important aspect of the present invention is the provision of a latent catalyst comprising at least one quaternary nitrogen compound as defined above. Such latent catalysts may find utility in any polymerization process, curable system or reactive system which may be catalyzed or co-catalyzed by tertiary amines. Without intention to limit the present invention, mention may be made of: curable systems containing an epoxy resin; curable systems comprising an isocyanate and an active hydrogen compound, such as an alcohol, a polyol, a thiol or a primary or secondary amine; the polymerization of ethylenically unsaturated monomers as described in US Patent No. 2,559,855 (Coover et al.); the Bayliss-Hillman coupling reaction; and, aldol condensation reactions.

In a particular embodiment, the present invention defines a polyurethane coating, adhesive or sealant composition comprising: a) a polyisocyanate; b) a polyol; and, c) a latent catalyst comprising at least one quaternary nitrogen compound as described herein before. As will be recognized by the skilled artisan, the polyisocyanate and the polyol should be present in such compositions in a pre-determined amount, selected to achieve the desired molar ratio of isocyanate (NCO) groups to hydroxyl groups (OH): that NCO: OH molar ratio should typically be in the range from 0.8:1 to 2.5:1, preferably from 1.3:1 to 1.8:1. The latent catalyst is desirably used in an amount of from 0.01 to 10 wt.%, and preferably from 0.01 to 5 wt.%, based on the total weight of the composition.

Such a polyurethane composition will desirably be a two component (2K) composition: the latent catalyst of the present invention may be disposed in one or both of the polyisocyanate (1^{st}) and polyol (2^{nd}) components. However, in a 2K composition, it is preferred that said latent catalyst be disposed exclusively in the polyol component (2^{nd}) of the composition.

In another interesting embodiment, the present invention defines a curable epoxy-resin composition for a coating, adhesive or sealant composition or as a matrix for composite materials, said epoxy resin composition comprising: a) an epoxy resin; b) a latent catalyst comprising at least one quaternary nitrogen compound as described herein before; and, optionally, c) a curative for said epoxy resin. The latent catalyst is desirably used in an amount of from 0.01 to 10 wt.%, and preferably from 0.01 to 5 wt.%, based on the total weight of the epoxy resin composition.

In certain embodiments, the epoxy resin compositions will comprise the epoxy resin and a curative in pre-determined amount; generally, the curative may be present in the composition in stoichiometric amounts ±50% relative to the epoxy resin component, with 80-100% of stoichiometry being preferred. For instance, where a curative contains active hydrogen atoms, the equivalence ratio of active hydrogen atoms relative to the epoxy groups in the resin should preferably be in the range from 1:0.5 to 1:1.5 and more preferably in the range from 1:0.8 to 1:1.2.

Whilst one component epoxy resin compositions of this type are not precluded, the compositions are desirably two-component compositions, with the first component containing the epoxy resin and the second component containing the curative and the latent catalyst.

There is no particular intention to limit the suitable curatives but specific mention may be made of: aliphatic polyamines, such as chain aliphatic polyamines, alicyclic polyamines and aliphatic aromatic polyamines; aromatic polyamines, such as metaphenylene diamine (MPDA), diaminodiphenyl methane (DDM) and diaminodiphenyl sulfone (DDS); amido amines; phenolics; thiols; and, polycarboxylic acids and anhydrides. And the disclosure of Lee et al. Handbook of Epoxy Resins, McGraw-Hill, pages 36-140, New York (1967) may be instructive in this regard.

As is standard in the art, curable compositions and, in particular, the above defined polyurethane and epoxy resin compositions may comprise additives and adjunct ingredients, provided these do not detrimentally affect the desired properties of the composition. Suitable additives and adjunct ingredients include: cocatalysts; antioxidants; UV absorbers/light stabilizers; metal deactivators; antistatic agents; reinforcers; fillers; antifogging agents; propellants; biocides; plasticizers; lubricants; emulsifiers; dyes; pigments; rheological agents; impact modifiers; adhesion regulators; optical brighteners; flame retardants; anti-drip agents; nucleating agents; wetting agents; thickeners; protective colloids; defoamers; tackifiers; solvents; reactive diluents; and, mixtures thereof. The selection of suitable conventional additives for the compositions of the invention depends on the specific intended use thereof and can be determined in the individual case by the skilled artisan.

Where employed, light stabilizers / UV absorbers, antioxidants and metal deactivators should preferably have a high migration stability and temperature resistance. They may suitable be selected, for example, from the groups a) to t) listed herein below, of which the compounds of groups a) to g) and i) represent light stabilizers/UV absorbers and compounds j) to t) act as stabilizers: a) 4,4-diarylbutadienes; b) cinnamic acid esters; c) benzotriazoles; d) hydroxybenzophenones; e) diphenyl cyanoacrylates; f) oxamides; g) 2-phenyl-1,3,5-triazines; h) antioxidants; i) nickel compounds; j) sterically hindered amines; k) metal deactivators; I) phosphites and phosphonites; m) hydroxylamines; n) nitrones; o) amine oxides; p) benzofuranones and indolinones; q) thiosynergists; r) peroxide-destroying compounds; s) polyamide stabilizers; and t) basic co-stabilizers.

The above defined polyurethane and epoxy resin compositions should comprise less than 5 wt.% of water, based on the weight of the composition, and are most preferably anhydrous compositions. These embodiments do not preclude the compositions from either comprising organic solvent or being essentially free of organic solvent. In an interesting embodiment, each said composition may be characterized by comprising, based on the weight of the composition less than 20 wt.%, preferably less than 10 wt.% of organic solvent.

Broadly, all organic solvents known to the person skilled in the art can be used as a solvent but it is preferred that said organic solvents are selected from the group consisting of: esters; ketones; halogenated hydrocarbons; alkanes; alkenes; and, aromatic hydrocarbons. Exemplary solvents are dichloromethane, trichloroethylene, toluene, xylene, butyl acetate, amyl acetate, isobutyl acetate, methyl isobutyl ketone, methoxybutyl acetate, cyclohexane, cyclohexanone, dichlorobenzene, diethyl ketone, di-isobutyl ketone, dioxane, ethyl acetate, ethylene glycol monobutyl ether acetate, ethylene glycol monoethyl acetate, 2-ethylhexyl acetate, glycol diacetate, heptane, hexane, isobutyl acetate, isooctane, isopropyl acetate, methyl ethyl ketone, tetrahydrofuran or tetrachloroethylene or mixtures of two or more of the recited solvents.

To form a composition - such as a coating, adhesive or sealant composition - the elements of the composition are brought together and mixed under conditions which inhibit or prevent activation of the latent catalyst. As such, it is preferred that the epoxy resin or polyisocyanate elements and the curative or active hydrogen bearing elements are not mixed by hand but are instead mixed by machine in pre-determined amounts under anhydrous conditions without intentional UV-irradiation. For instance, for small-scale applications in which volumes of less than 1 liter will generally be used, the composition may be formed by co-extrusion of the elements of the composition from a plurality of side-by-side double or coaxial cartridges through a closely mounted static or dynamic mixer. For larger applications, the elements of the composition may be supplied via pipelines to a mixing apparatus which can ensure fine and highly homogeneous mixing of *inter alia* the latent catalyst and the reactive elements in the absence of moisture and under limited irradiation.

The curable compositions according to the invention should have a viscosity in mixed form of 100 to 3000 mPa.s, preferably 100 to 1500 mPa.s, as measured at a temperature of 20°C and determined immediately after mixing, for example, up to two minutes after mixing.

The compositions of the present invention may be applied to a given substrate by conventional application methods such as: brushing; roll coating using, for example, a 4-application roll equipment where the composition is solvent-free or a 2-application roll equipment for solvent-containing compositions; doctor-blade application; printing methods; and, spraying methods, including but not limited to air-atomized spray, air-assisted spray, airless spray and high-volume low-pressure spray. For coating and adhesive applications, it is recommended that the compositions be applied to a wet film thickness of from 10 to 500 µm. The application of thinner layers within this range is more economical and provides for a reduced likelihood of thick cured regions that may - for coating applications - require sanding. However, great control must be exercised in applying thinner coatings or layers so as to avoid the formation of discontinuous cured films.

Subsequent to their application, the compositions according to the present invention may typically be activated in less than a minute, and commonly between 1 and 20 seconds - for instance between 3 and 12 seconds - when irradiated using commercial UV curing equipment. Before UV activation they additionally demonstrate a long pot life, typically of at least 60 minutes and commonly of at least 90 or 120 minutes. The pot life shall be understood herein to be the time after which the viscosity of a mixture at 20°C will have risen to more than 50,000 mPas.

The irradiating ultraviolet light - acting as an external stimulus for the latent catalyst - should typically have a wavelength of from 150 to 600 nm and preferably a wavelength of from 200 to 450 nm. Useful sources of UV light include, for instance, extra high pressure mercury lamps, high pressure mercury lamps, medium pressure mercury lamps, low intensity fluorescent lamps, metal halide lamps, microwave powered lamps, xenon lamps, UV-LED lamps and laser beam sources such as excimer lasers and argon-ion lasers.

The amount of radiation necessary to cure an individual composition will depend on a variety of factors including the angle of exposure to the radiation, the thickness of a coating layer and the volume of a mold where, for instance, the composition is to be employed as a matrix of a composite material. Broadly however, a curing dosage of from 5 to 5000 mJ/cm² may be cited as being typical: curing dosages of from 50 to 500 mJ/cm², such as from 50 to 400 mJ/cm² may be considered highly effective.

The curing or hardening of the epoxy resin or polyurethane compositions of the invention typically occurs at temperatures in the range of from -10°C to 150°C, preferably from 0°C to 100°C, and in particular from 10°C to 70°C. The temperature that is suitable depends on the specific curatives and the desired hardening rate and can be determined in the individual case by the skilled artisan, using simple preliminary tests if necessary. Of course, curing at temperatures of from 5°C to 35°C or from 20°C to 30°C is especially advantageous as it obviates the requirement to substantially heat or cool the compositions from the usually prevailing ambient temperature. Where applicable, however, the temperature of the composition may be raised prior to, during or after application using conventional means.

The epoxy resin or polyurethane compositions according to the invention may find utility *inter alia* in: varnishes; inks; elastomers; foams; binding agents for particles and / or fibers, such as carbon fibers; the coating of glass; the coating of ceramic; the coating of mineral building materials, such as mortar, brick, tile, natural stone, lime- and / or cement-bonded plasters, gypsum-containing surfaces, fiber cement building materials and concrete; the coating and sealing of wood and wooden materials, such as chipboard, fiber board and paper; the coating of metallic surfaces; the coating of asphalt- and bitumen-containing pavements; the coating and sealing of various plastic surfaces; and, the coating of leather and textiles.

It is also considered that the compositions of the present invention are suitable as pourable sealing compounds for electrical building components such as cables, fiber optics, cover strips or plugs. The sealants may serve to protect those components against the ingress of water and other contaminants, against heat exposure, temperature fluctuation and thermal shock, and against mechanical damage.

The compositions are equally suitable for forming composite structures by surface-to-surface bonding of the same or different materials to one another. The binding together of wood and wooden materials and the binding together of metallic materials may be mentioned as exemplary adhesive applications of the present compositions.

In a particularly preferred embodiment of the invention, the epoxy or polyurethane compositions are used as solvent-free or solvent-containing lamination adhesives The present invention thus also provides a method of forming a flexible film laminate, said method comprising the steps of: a) providing first and second flexible films; b) providing a curable composition as defined hereinabove; c) disposing the curable composition on at least a portion of one surface of the first flexible film; d) joining the first flexible film and a second flexible film so that the curable adhesive mixture is interposed between the first flexible film and the second flexible film; and, e) simultaneously with or subsequent to said joining step, irradiating said adhesive mixture with ultraviolet light to cure said mixture.

The first flexible film and a second flexible film are joined so that the curable adhesive composition is interposed between the first flexible film and the second flexible film. Depending on the films used in a lamination, the UV radiation necessary to cure the adhesive mixture can be passed through one or both films or, conversely, it may be necessary to irradiate the adhesive mixture when it is accessible in the joining step, that is prior to and during the mating of the film surfaces.

In the packaging applications for which the present invention is envisaged to be particularly suitable, the first and second flexible films will typically be constituted by polyethylene, polyester, polyethylene terephthalate, oriented polypropylene, ethylene vinylacetate, poly(methylmethacrylate), polycarbonate (PC), Acrylonitrile Butadiene Styrene (ABS), co-extruded films, metal foils and the like. Obviously, if the irradiation step e) is be performed subsequent to said joining step, this restricts the films used to ones that are UV transparent. Oriented polypropylene has proven to be an excellent film in this regard because it absorbs ≤ 10% of UV radiation. In contrast, polyethylene terephthalate based films absorb c. 40% of the UV radiation from an H-bulb and are not practical candidates for the curing of the adhesives of the present invention by UV irradiation of the adhesives through the film.

The following examples are illustrative of the present invention, and are not intended to limit the scope of the invention in any way.

### EXAMPLES

The following materials are used in the Examples:
- LIOFOL LA 6707:: Polyester Polyol having an OH number of ca.135 mgKOH/g and an Mw of ca. 2600 g/mol, available from Henkel AG & Co. KGaA.
- LIOFOL LA 7707:: NCO-pre-polymer (polyisocyanate) based on MDI, polyether polyols and castor oil, available from Henkel AG & Co. KGaA.
- D.E.R.331™: Liquid Epoxy Resin, a reaction product of epichlorohydrin and bisphenol A, available from The Dow Chemical Company.

### Example 1: Synthesis of PEG-dilm-Ant-BPh₄

This Example relates to the multi-stage synthesis of the following quaternary nitrogen compound (above identified as PEG-dilm-Ant-BPh₄).

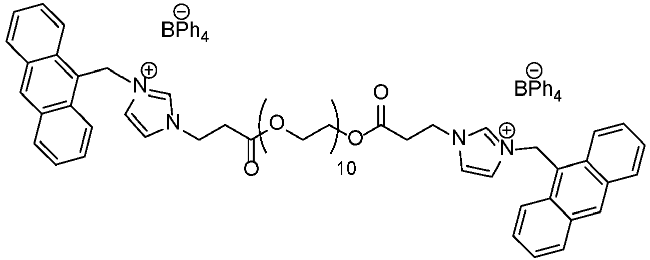

### Stage 1.1: Synthesis of PEG-dilm

In a 1-necked 100 mL flask equipped with a magnetic bar, 8.6 g of poly(ethylene glycol) diacrylate (12.3 mmol), 50 mL of chloroform and 1.8 g of imidazole (27.0 mmol) were introduced. The mixture was heated up to 80 °C and left, under stirring, for 48 hours. The solvent was then evaporated under reduced pressure. The crude product obtained was dissolved in the minimum amount of dichloromethane (∼10 mL) and precipitated in 100 mL of diethyl ether at 0 °C three times. The light orange oil obtained was isolated and dried under reduced pressure.

### Stage 1.2: Synthesis of PEG-dilm-Ant-Cl

In a 2-necked 50 mL flask equipped with a magnetic bar, 0.70 g of 9-chloromethyl anthracene (3.10 mmol), 10 mL of dichloromethane and 1.0 g of PEG-dilm from Stage 1.1 (1.41 mmol) were introduced under flux of argon. The mixture was left, under stirring, at room temperature for 48 hours. The solvent was then evaporated under reduced pressure. The crude product obtained was dissolved in the minimum amount of dichloromethane (∼1 mL) and precipitated in 20 mL of diethyl ether at 0 °C three times. The brown oil obtained was isolated and dried under reduced pressure.

### Stage 1.3: Synthesis of PEG-dilm-Ant-BPh₄

In a 1-necked 50 mL flask equipped with a magnetic bar, 0.40 g of PEG-dilm-Ant-Cl from Stage 1.2 (0.32 mmol) was dissolved in 10 mL of methanol. Then, 0.22 g of sodium tetraphenyl borate (0.64 mmol), previously dissolved in the minimum amount of methanol (∼1 mL), was added dropwise to the flask. A precipitate was formed immediately and the mixture was left, under stirring, at room temperature overnight. The solid was isolated by filtration and rinsed with methanol several times. The orange pale powder obtained was dried under reduced pressure. The overall yield of this powder was 56%.

### Example 2: Synthesis of PEG-Im-Ant-BPh₄

This Example relates to the multi-stage synthesis of the following compound (above identified as PEG-Im-Ant-BPh₄).

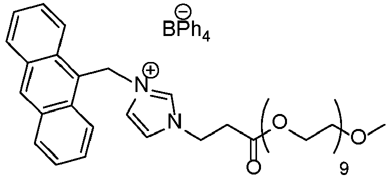

### Stage 2.1: Synthesis of PEG-Im

In a 3-necked 250 mL flask equipped with a magnetic bar, 36 g of poly(ethylene glycol) methyl ether acrylate (75.0 mmol), 90 mL of chloroform and 5.1 g of imidazole (75.0 mmol) were introduced. The mixture was heated up to 80 °C and left, under stirring, for 48 hours. The solvent was then evaporated under reduced pressure. The crude product obtained was dissolved in the minimum amount of dichloromethane (∼40 mL) and precipitated in 400 mL of diethyl ether at 0 °C three times. The light orange oil obtained was isolated and dried under reduced pressure.

### Stage 2.2: Synthesis of PEG-Im-Ant-CI

In a 3-necked 250 mL flask equipped with a magnetic bar, 9.1 g of 9-chloromethyl anthracene (40.1 mmol), 30 mL of dimethylformamide and 22 g of PEG-Im from Stage 2.1 (40.1 mmol) were introduced under flux of argon. The mixture was subsequently heated up to 70 °C and left overnight under stirring. The mixture was used without further purification in the next stage (2.3): it could, alternatively, have been precipitated in 400 mL of diethyl ether at 0 °C three times.

### Stage 2.3: Synthesis of PEG-Im-Ant-BPh₄

The reaction mixture of Stage 2.2 (PEG-Im-Ant-CI) was cooled down to room temperature and 30 mL of methanol were added. Then, 13.7 g of sodium tetraphenyl borate (40.1 mmol), previously dissolved in the minimum amount of methanol (∼15 mL), was added dropwise to the flask. The reaction mixture was heated up to 70°C for 4 hours and, after subsequent cooling, was left at room temperature overnight under stirring. The solvent was then evaporated under reduced pressure and the crude product was dissolved in tetrahydrofuran. The precipitated salts were removed by filtration through diatomaceous earth and a transparent solution was obtained. After removing a portion of the solvent such that the product was dissolved in the minimum amount of tetrahydrofuran, the product was precipitated in 400 mL of diethyl ether and the brown oil obtained was dried under reduced pressure. The overall yield of this oil was 80%.

### Example 3: Synthesis of PPG-b-PEG-diGUA-Ant-BPh4

This Example relates to the multi-stage synthesis of the following compound (above identified as PPG-b-PEG-diGUA-Ant-BPh₄).

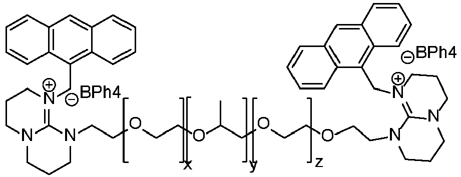

### Stage 3.1: Synthesis of PPG-b-PEG-diGUA

In a 3-necked 250 mL flask equipped with a magnetic bar, 35 g of poly(ethylene glycol)-block-poly(propylene glycol)-block-poly(ethylene glycol) (17.5 mmol), 80 mL of Toluene and 5.3 g of anhydrous triethylamine (52.5 mmol) were introduced. Then, 7.3 g of p-Toluenesulfonyl chloride (38.5 mmol) was added to the flask, the reaction mixture was left to stir at room temperature for 30 min and then heated up to 60 °C for 48 hours. Once the reaction was finished -the progress of the reaction being followed by NMR - the mixture was allowed to cool down to room temperature. The cooled mixture was added, via cannula equipped with filter paper, to a 3-necked 250 mL flask containing 4 g of KOH (70 mmol), 5 g of 1,5,7-Triazabicyclo[4.4.0]dec-5-ene (35 mmol) and 40 mL of anhydrous dimethylformamide. The reaction mixture was left overnight at 80 °C under stirring.

### Stage 3.2: Synthesis of PPG-b-PEG-diGUA-Ant-Cl

In a 3-necked 250 mL flask equipped with a magnetic bar, 34 g of PPG-b-PEG-diGUA from Stage 3.1 (30 mmol), 40 mL of dimethylformamide and 7.1 g of 9-chloromethyl anthracene were introduced. The mixture was left to stir overnight at 60 °C and, after being cooled down to room temperature, it was used without further purification in the next stage (3.3).

### Stage 3.3: Synthesis of PPG-b-PEG-diGUA-Ant-BPh₄

To the flask containing the product obtained in Stage 3.2 (PPG-b-PEG-diGUA-Ant-CI), 40 mL of methanol was added. Then, 10.3 g of sodium tetraphenyl borate (30 mmol), previously dissolved in the minimum amount of methanol (∼10 mL), was added dropwise to the flask. The reaction mixture was heated up to 70°C for 4 hours and, after cooling down, was left at room temperature overnight under stirring. The solvent was evaporated under reduced pressure and the crude product was dissolved in tetrahydrofuran. The precipitated salts were removed by filtration through diatomaceous earth and a transparent solution was obtained. After removing the solvent, the product was precipitated in 400 mL of diethyl ether and the brown oil obtained was dried under reduced pressure. The overall yield of this oil was 62%.

### Example 4: Synthesis of PPG-b-PEG-dilm-Ant-BPh4

This Example relates to the multi-stage synthesis of the following compound (above identified PPG-b-PEG-dilm-Ant-BPh4).

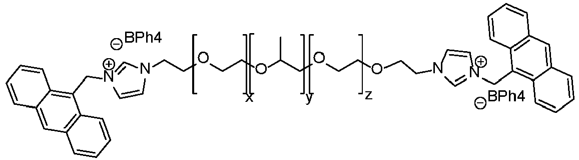

### Stage 4.1: Synthesis of PPG-b-PEG-dilm

In a 3-necked 250 mL flask equipped with a magnetic bar, 35 g of poly(ethylene glycol)-block-poly(propylene glycol)-block-poly(ethylene glycol) (17.5 mmol), 100 mL of dichloromethane and 7.1 g of anhydrous triethylamine (70 mmol) were introduced. Then, the reaction was cooled down at 0 °C in an ice bath and 3.80 g of acryloyl chloride (42 mmol) was slowly added to the flask. The reaction mixture was left to stir at 0 °C for 30 minutes and then left at room temperature for 2 hours. Once the reaction was finished (as monitored by NMR), the ammonium salt precipitated was filtered off and the filtrate was washed with 1 M NaOH solution (three times), with a saturated solution of NaHCO₃ (three times) and with brine (three times). The final solution was dried with MgSO₄ and the solvent was evaporated under reduced pressure.

The crude product thus obtained was dissolved in 100 mL of chloroform in a 250 mL round bottom flask and 2.5 g of imidazole (36.8 mmol) was added. The mixture was heated at 80 °C overnight. After that, the crude product was used in the next step without further purification.

### Stage 4.2: Synthesis of PPG-b-PEG-dilm-Ant-Cl

To the 250 mL round bottom flask containing the reaction mixture from stage 4.1 (PPG-b-PEG-dilm), 35 mL of dimethylformamide and 7.5 g of 9-chloromethyl anthracene (33 mmol) were added. The mixture was left to stir overnight at 60 °C and, after cooling down to room temperature, the mixture was used without further purification in the next stage (4.3).

### Stage 4.3: Synthesis of PPG-b-PEG-dilm-Ant-BPh₄

To the flask containing the product obtained in stage 5.2 (PPG-b-PEG-dilm-Ant-CI), 35 mL of methanol was added. Then, 12 g of sodium tetraphenyl borate (35 mmol), previously dissolved in the minimum possible amount of methanol (∼10 mL), was added dropwise to the flask. The reaction mixture was heated up to 70°C for 4 hours and, after subsequent cooling down, was left at room temperature overnight under stirring. The solvent was evaporated under reduced pressure and the crude product was dissolved in tetrahydrofuran. The precipitated salts were removed by filtration through diatomaceous earth and a transparent solution was obtained. After removing the solvent, the product was precipitated in 400 mL of diethyl ether and the brown oil obtained was dried under reduced pressure. The overall yield of this oil was 45%.

In the below mentioned Example 5, the progress of the described curing reactions was monitored using Fourier transform infrared spectroscopy (FTIR) in conjunction with Attenuated Total Reflection (ATR), hereinafter FTIR (ATR). For each FTIR absorption spectrum, the carbonyl peak was identified at around 1700 cm⁻¹ whilst the disappearance of the isocyanate peak at around 2270 cm⁻¹ was monitored. [The absorption peak of the carbonyl group does shift to a slightly higher wavenumber as the polyisocyanate content of the adhesive mixture declines.] Specifically, the ratio of the area of the peak at 2270 cm⁻¹ to the peak at around 1700 cm⁻¹ was quantified, the decline of which quantity over time correlates to the consumption of the polyisocyanate in the polyaddition reaction.

### Example 5: Two Component (2K) Polyurethane Composition

1.5 g of polyol (LA 6707) was mixed with 45 mg of PEG-b-PPG-dilm-Ant-BPh₄ (Example 4) diluted with 2 mL DCM. In addition, 2 g of isocyanate (LA 7707) was also diluted with dichloromethane and then mixed with the previous solution. The resulting adhesive mixture was placed on an aluminium foil and spread with a coating bar (No. 4). The solvent was allowed to evaporate and the coating heated for 30 seconds at 50°C with a heat gun. The coating average over the aluminium foil was around 9 g/m². The coated aluminium foil sample was designated as CF1.

For comparative analysis, a further portion of the above adhesive mixture was prepared but without the added latent catalyst. This adhesive mixture was similarly applied to aluminium foil and the resultant coated aluminium foil sample was designated as CF2.

The coated aluminium foil samples were then permitted to cure under the following conditions:
CF1: UV irradiation at a curing dosage of 300 mJ/cm²
CF2: Ambient conditions without irradiation

The results of the FTIR (ATR) monitoring of the curing of the adhesive mixtures are shown in Table 1 herein below.

**Table 1**

| CF1 | | CF2 | |
|---|---|---|---|
| Time (min) | **Peak Ratio** | Time (min) | **Peak Ratio** |
| 7 | **10.6** | 6 | **12.1** |
| 10 | **10.0** | 9 | **12.1** |
| 27 | **7.2** | 26 | **10.9** |
| 35 | **6.7** | 35 | **10.7** |
| 53 | **5.6** | 52 | **9.9** |
| 68 | **4.8** | 68 | **9.0** |
| 108 | **4.3** | 107 | **7.8** |
| 145 | **3.3** | 145 | **7.3** |

### Example 6: Two Component (2K) Epoxy Thiol Curable Composition

In a glass vial were mixed 1.00 g of epoxy resin (D.E.R 331), 0.50 g of thiol-based hardener (1,8-Dimercapto-3,6-Dioxaoctane, DMDO) and 50 mg of latent catalyst from Example 3 (PEG-b-PPG-diGUA-Ant-BPh4). The mixture was homogenized by stirring for 1 minute with a spatula.

To study the activity and latency of the catalyst in the mixture, five samples from the mixture were treated in the following manner:
S1: The sample was irradiated for 5 seconds with UV light
S2: The sample was irradiated for 20 seconds with UV light
S3: The sample was post-cured at 80 °C for 1 hour after 5 seconds of irradiation
S4: The sample was post-cured at 80 °C for 1 hour without irradiation
S5: The sample was neither irradiated nor thermally treated

The curing profiles of the treated samples were studied by Differential Scanning Calorimetry (DSC) in order to determine the efficacy of the latent catalyst. The following DSC cycles were used: i) 1^{st} Heating from -20 °C to 250 °C at 10 °C/min; ii) Cooling from 250 °C to -20 °C at 10 °C/min; and, iii) 2^{nd} Heating from -20 °C to 250 °C at 10 °C/min.

The curing profiles are described in Table 2 herein below of which profiles the respective glass transition temperatures (Tg) were determined from the 2^{nd} heating cycle.

**Table 2**

| **Resin System** | **Sample** | **Peak Max. (°C)** | **ΔH (J/q)** | **Tq* (°C)** |
|---|---|---|---|---|
| DER-DMDO | S1 | 137 | 460 | 3.4 |
| | S2 | 135 | 466 | 6.7 |
| | S3 | - | - | 10 |
| | S4 | 179 | 376 | 10.7 |
| | S5 | 187 | 459 | 10.0 |

## Claims

1. A quaternary nitrogen compound comprising a nitrogen heterocycle wherein:
at least one polymeric substituent is bound to a ring atom of said heterocycle; and,
an aromatic photoremovable group (PRG) is directly bound to a quaternary nitrogen ring atom of said heterocycle, the release of said aromatic photoremovable group under UV irradiation yielding a tertiary amine, in which said aromatic photoremovable group (PRG) is represented by the formula:
-CR¹R²-Ar (PRG)
wherein: R¹ and R² are independently of one another hydrogen or C1-C6 alkyl;
Ar represents an aryl group having from 9 to 18 ring carbon atoms, which aryl group may be unsubstituted or may be substituted by one of more C1-C6 alkyl group, C2-C4 alkenyl group, CN, OR³, SR³, CH₂OR³, C(O)R³, C(O)OR³ or halogen; and,
where present, each R³ is independently selected from the group consisting of hydrogen, C1-C6-alkyl and phenyl,
in which the at least one polymeric substituent is selected from polyethers, polyesters, polycarbonates, and copolymers thereof, and
the nitrogen heterocycle is an unsaturated monocyclic or bicyclic nitrogen heterocycle having at least two ring nitrogen atoms.

2. The compound according to claim 1 comprising a stoichiometric amount of a counter ion of anionic charge selected from halides and non-coordinating anions comprising an element selected from boron, phosphorous or silicon.

3. The compound according to claim 1, wherein the Ar represents an aryl group selected from phenanthrenyl, anthracenyl, fluorenyl, and indenyl.

4. The compound according to claim 1, wherein:
at least one of R¹ and R² is hydrogen; and
Ar represents an aryl group having from 9 to 18 ring carbon atoms, which aryl group may be unsubstituted or may be substituted by one or more C1-C6 alkyl group, C2-C4 alkenyl group, C(O)R³ or halogen.

5. The compound according to any one of claims 1 to 4, wherein said compound is denoted by the general formula:
Y-(L-β-PRG)ᵣ
wherein: Y is an r-valent polymeric radical selected from the group consisting of polyethers, polyesters, polycarbonates, and copolymers thereof;
L is a covalent bond or an organic linking group;
β represents a nitrogen heterocycle having a quaternary nitrogen ring atom with which is associated a charge balancing anion;
PRG is said aromatic photoremovable group bound to a quaternary nitrogen ring atom of said heterocycle, in which said aromatic photoremovable group (PRG) is represented by the formula:
-CR¹R²-Ar (PRG)
wherein: R¹ and R² are independently of one another hydrogen or C1-C6 alkyl;
Ar represents an aryl group having from 9 to 18 ring carbon atoms, which aryl group may be unsubstituted or may be substituted by one of more C1-C6 alkyl group, C2-C4 alkenyl group, CN, OR³, SR³, CH₂OR³, C(O)R³, C(O)OR³ or halogen; and,
where present, each R³ is independently selected from the group consisting of hydrogen, C1-C6-alkyl and phenyl; and,
r is an integer of at least 1, preferably an integer of from 1 to 4.

6. The compound according to claim 5 wherein the group -β-PRG is represented by either: 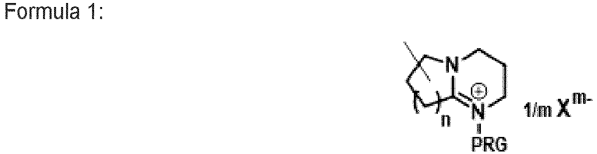 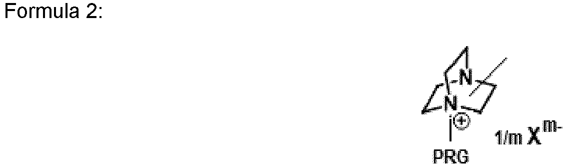 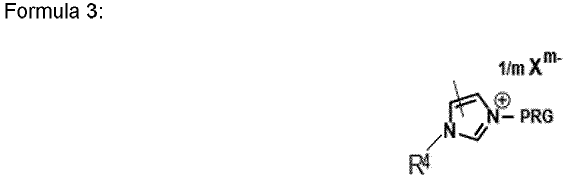 or, 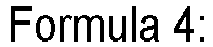 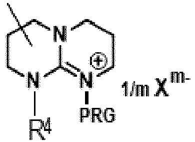
wherein: n is 1, 2 or 3;
R⁴ is hydrogen, C1-C6 alkyl, phenyl or a polymeric substituent which is represented by the general formula -L˙Y in which L˙ is a covalent bond or an organic linking group and Y˙ is a polymeric radical selected from the group consisting of polyethers, polyesters, polycarbonates, and copolymers thereof; and,
X^{m-} is a counter ion of anionic charge m selected from halides and non-coordinating anions comprising an element selected from boron, phosphorous or silicon.

7. The compound according to claim 5 or claim 6, wherein said r-valent polymeric radical Y is a polyether selected from group consisting of polyalkylene oxides and copolymers thereof.

8. The compound according to claim 7, wherein said r-valent polymeric radical Y is either a linear homopolymer of ethylene oxide or propylene oxide or a linear copolymer of ethylene oxide, propylene oxide and, optionally, butylene oxide.

9. The compound according to any one of claims 5 to 8, wherein said r-valent polymeric radical Y is **characterized by** a weight average molecular weight of from 100 to 100,000, preferably 400 to 7500 g/mol.

10. Use of the compound as defined in any one of claims 1 to 9 as a latent catalyst in a curable composition, the curing of which may be catalyzed or co-catalyzed by tertiary amines.

11. The use as defined in claim 10 **characterized in that** said compound is irradiated with ultraviolet light having:
a wavelength of from 150 to 600 nm, preferably from 200 to 450 nm; and / or
an energy of from 5 to 500 mJ/cm², preferably from 50 to 400 mJ/cm².

12. A polyurethane coating, adhesive or sealant composition comprising:
a) a polyisocyanate;
b) a polyol; and,
c) a latent catalyst comprising at least one compound as defined in any one of claims 1 to 9.

13. A curable epoxy-resin composition for a coating, adhesive or sealant composition or for a matrix for composite materials, said epoxy resin composition comprising:
a) an epoxy resin;
b) a latent catalyst comprising at least one compound as defined in any one of claims 1 to 9; and, optionally
c) a curative for said epoxy resin.

14. The composition according to claim 12 or claim 13 comprising said latent catalyst in an amount of from 0.01 to 10 wt.%, preferably from 0.01 to 5 wt.%, based on the total weight of the composition.

## Patentansprüche

1. Quartäre Stickstoffverbindung, umfassend einen Stickstoffheterocyclus, wobei:
mindestens ein polymerer Substituent an ein Ringatom des Heterocyclus gebunden ist; und
eine aromatische photoentfernbare Gruppe (photoremovable group - PRG) direkt an ein quaternäres Stickstoffringatom des Heterocyclus gebunden ist, wobei die Freisetzung der aromatischen photoentfernbaren Gruppe unter UV-Bestrahlung ein tertiäres Amin ergibt, wobei die aromatische photoentfernbare Gruppe (PRG) durch die Formel dargestellt wird:
-CR¹R²-Ar (PRG)
wobei: R¹ und R² unabhängig voneinander Wasserstoff oder C1-C6-Alkyl sind;
Ar eine Arylgruppe darstellt, die 9 bis 18 Ringkohlenstoffatome aufweist, wobei die Arylgruppe unsubstituiert sein kann oder durch eins von mehreren C1-C6-Alkylgruppen, C2-C4-Alkenylgruppen, CN, OR³, SR³, CH₂OR³, C(O)R³, C(O)OR³ oder Halogen substituiert sein kann; und
falls vorhanden, jedes R³ unabhängig aus der Gruppe ausgewählt ist, die aus Wasserstoff, C1-C6-Alkyl und Phenyl besteht,
wobei der mindestens eine polymere Substituent aus Polyethern, Polyestern, Polycarbonaten und Copolymeren davon ausgewählt ist, und
der Stickstoffheterocyclus ein ungesättigter monocyclischer oder bicyclischer Stickstoffheterocyclus ist, der mindestens zwei Ringstickstoffatome aufweist.

2. Verbindung nach Anspruch 1, umfassend eine stöchiometrische Menge eines Gegenions mit anionischer Ladung, das aus Halogeniden und nicht-koordinierenden Anionen ausgewählt ist, umfassend ein Element, das aus Bor, Phosphor oder Silicium ausgewählt ist.

3. Verbindung nach Anspruch 1, wobei das Ar eine Arylgruppe darstellt, die aus Phenanthrenyl, Anthracenyl, Fluorenyl und Indenyl ausgewählt ist.

4. Verbindung nach Anspruch 1, wobei:
R¹ und/oder R² Wasserstoff ist; und
Ar eine Arylgruppe darstellt, die 9 bis 18 Ringkohlenstoffatome aufweist, wobei die Arylgruppe unsubstituiert sein kann oder durch eine oder mehrere C1-C6-Alkylgruppen, C2-C4-Alkenylgruppen, C(O)R³ oder Halogen substituiert sein kann.

5. Verbindung nach einem der Ansprüche 1 bis 4, wobei die Verbindung durch die allgemeine Formel bezeichnet wird:
Y-(L-β-PRG)ᵣ
wobei: Y ein r-wertiger polymerer Rest ist, der aus der Gruppe ausgewählt ist, die
aus Polyethern, Polyestern, Polycarbonaten und Copolymeren davon besteht;
L eine kovalente Bindung oder eine organische Verknüpfungsgruppe ist;
β einen Stickstoffheterocyclus darstellt, der ein quaternäres Stickstoffringatom aufweist, dem ein ladungsausgleichendes Anion zugehörig ist;
PRG die aromatische photoentfernbare Gruppe ist, die an ein quaternäres Stickstoffringatom des Heterocyclus gebunden ist, wobei die aromatische photoentfernbare Gruppe (PRG) durch die Formel dargestellt wird:
-CR¹R²-Ar (PRG)
wobei: R¹ und R² unabhängig voneinander Wasserstoff oder C1-C6-Alkyl sind;
Ar eine Arylgruppe darstellt, die 9 bis 18 Ringkohlenstoffatome aufweist, wobei die Arylgruppe unsubstituiert sein kann oder durch eins von mehreren C1-C6-Alkylgruppen, C2-C4-Alkenylgruppen, CN, OR³, SR³, CH₂OR³, C(O)R³, C(O)OR³ oder Halogen substituiert sein kann; und
falls vorhanden, jedes R³ unabhängig aus der Gruppe ausgewählt ist, die aus Wasserstoff, C1-C6-Alkyl und Phenyl besteht; und
r eine ganze Zahl von mindestens 1, vorzugsweise eine ganze Zahl von 1 bis 4, ist.

6. Verbindung nach Anspruch 5, wobei die Gruppe-ß-PRG dargestellt wird durch entweder: 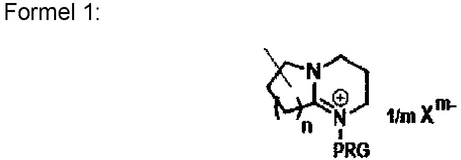 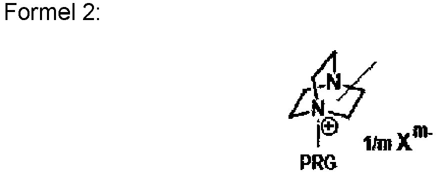 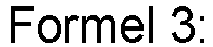 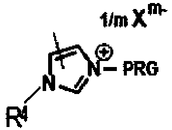 oder 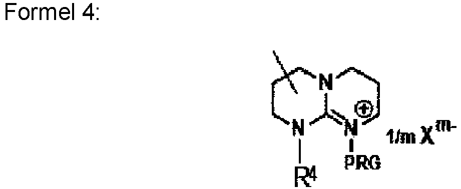
wobei: n 1, 2 oder 3 ist.
R⁴ Wasserstoff, C1-C6-Alkyl, Phenyl oder ein polymerer Substituent ist, der durch die allgemeine Formel -L˙ Y dargestellt wird, wobei L˙ eine kovalente Bindung oder eine organische Verknüpfungsgruppe ist und Y˙ ein polymerer Rest ist, der aus der Gruppe ausgewählt ist, die aus Polyethern, Polyestern, Polycarbonaten und Copolymeren davon besteht; und
X^{m-} ein Gegenion mit anionischer Ladung m ist, das aus Halogeniden und nicht-koordinierenden Anionen ausgewählt ist, umfassend ein Element, das aus Bor, Phosphor oder Silicium ausgewählt ist.

7. Verbindung nach Anspruch 5 oder 6, wobei der r-wertige polymere Rest Y ein Polyether ist, der aus der Gruppe ausgewählt ist, die aus Polyalkylenoxiden und Copolymeren davon besteht.

8. Verbindung nach Anspruch 7, wobei der r-wertige polymere Rest Y entweder ein lineares Homopolymer von Ethylenoxid oder Propylenoxid oder ein lineares Copolymer von Ethylenoxid, Propylenoxid und optional Butylenoxid ist.

9. Verbindung nach einem der Ansprüche 5 bis 8, wobei der r-wertige polymere Rest Y durch ein Massenmittel von 100 bis 100.000, vorzugsweise 400 bis 7500 g/mol, gekennzeichnet ist.

10. Verwendung der Verbindung nach einem der Ansprüche 1 bis 9 als ein latenter Katalysator in einer härtbaren Zusammensetzung, deren Härten durch tertiäre Amine katalysiert oder co-katalysiert werden kann.

11. Verwendung nach Anspruch 10, **dadurch gekennzeichnet, dass** die Verbindung mit ultraviolettem Licht bestrahlt wird, aufweisend:
eine Wellenlänge von 150 bis 600 nm, vorzugsweise von 200 bis 450 nm; und/oder
eine Energie von 5 bis 500 mJ/cm², vorzugsweise von 50 bis 400 mJ/cm².

12. Polyurethanbeschichtungs-, Klebstoff- oder Dichtungsmaterialzusammensetzung, umfassend:
a) ein Polyisocyanat;
b) ein Polyol; und
c) einen latenten Katalysator, umfassend mindestens eine Verbindung nach einem der Ansprüche 1 bis 9.

13. Härtbare Epoxidharzzusammensetzung für eine Beschichtungs-, Klebstoff- oder Dichtungsmaterialzusammensetzung oder für eine Matrix für Verbundwerkstoffmaterialien, wobei die Epoxidharzzusammensetzung umfasst:
a) ein Epoxidharz,
b) einen latenten Katalysator, umfassend mindestens eine Verbindung nach einem der Ansprüche 1 bis 9; und optional
c) ein Härtungsmittel für das Epoxidharz.

14. Zusammensetzung nach Anspruch 12 oder 13, umfassend den latenten Katalysator in einer Menge von 0,01 bis 10 Gew.-%, vorzugsweise von 0,01 bis 5 Gew.-%, basierend auf dem Gesamtgewicht der Zusammensetzung.

## Revendications

1. Composé azoté quaternaire comprenant un hétérocycle azoté, où :
au moins un substituant polymère est lié à un atome de noyau dudit hétérocycle ; et,
un groupe photoamovible aromatique (PRG) est directement lié à un atome de noyau azoté quaternaire dudit hétérocycle, la libération dudit groupe photoamovible aromatique sous exposition à un rayonnement UV donnant une amine tertiaire, dans lequel ledit groupe photoamovible aromatique (PRG) est représenté par la formule :
-CR¹R²-Ar (PRG)
où : R¹ et R² sont indépendamment soit de l'hydrogène, soit un alkyle en C₁ à C₆;
Ar représente un groupe aryle ayant de 9 à 18 atomes de carbone de noyau, lequel groupe aryle peut être non substitué ou peut être substitué par un ou plusieurs groupe alkyle en C₁ à C₆, groupe alcényle en C₂ à C₄, CN, OR³, SR³, CH₂OR³, O(O)R³, C(O)OR³ ou halogène ; et,
s'il est présent, chaque R³ est choisi indépendamment dans le groupe constitué d'hydrogène, alkyle en C₁ à C₆ et phényle,
dans lequel ledit substituant polymère est choisi parmi les polyéthers, polyesters, polycarbonates et leurs copolymères, et
l'hétérocycle azoté est un hétérocycle azoté monocyclique ou bicyclique insaturé comportant au moins deux atomes d'azote de noyau.

2. Composé selon la revendication 1 comprenant une quantité stoechiométrique d'un contre-ion de charge anionique choisi parmi les halogénures et les anions non coordinants comprenant un élément choisi parmi le bore, le phosphore ou le silicium.

3. Composé selon la revendication 1, où Ar représente un groupe aryle choisi parmi phénanthrényle, anthracényle, fluorényle et indényle.

4. Composé selon la revendication 1, où :
R¹ et/ou R² est de l'hydrogène ; et
Ar représente un groupe aryle ayant de 9 à 18 atomes de carbone de noyau, lequel groupe aryle peut être non substitué ou peut être substitué par un ou plusieurs groupe alkyle en C₁ à C₆, groupe alcényle en C₂ à C₄, C(O)R³ ou halogène.

5. Composé selon l'une quelconque des revendications 1 à 4, où ledit composé est désigné par la formule générale :
Y-(L-β-PRG)ᵣ
où : Y est un radical polymère r-valent choisi dans le groupe constitué de polyéthers, polyesters, polycarbonates et leurs copolymères ;
L représente une liaison covalente ou un groupe de jonction organique ;
β représente un hétérocycle azoté comportant un atome de noyau d'azote quaternaire auquel est associé un anion d'équilibrage de charge ;
PRG est ledit groupe photoamovible aromatique lié à un atome de noyau d'azote quaternaire dudit hétérocycle, où ledit groupe photoamovible aromatique (PRG) est représenté par la formule :
-CR¹R²-Ar (PRG)
où : R¹ et R² sont indépendamment soit de l'hydrogène, soit un alkyle en C₁ à C₆;
Ar représente un groupe aryle ayant de 9 à 18 atomes de carbone de noyau, lequel groupe aryle peut être non substitué ou peut être substitué par un ou plusieurs groupe alkyle en C₁ à C₆, groupe alcényle en C₂ à C₄, CN, OR³, SR³, CH₂OR³, C(O)R³, C(O)OR³ ou halogène ; et,
s'il est présent, chaque R³ est indépendamment choisi dans le groupe constitué d'hydrogène, alkyle en C₁ à C₆ et phényle ; et,
r est un nombre entier d'au moins 1, de préférence un nombre entier de 1 à 4.

6. Composé selon la revendication 5, où le groupe -β-PRG est représenté soit par : 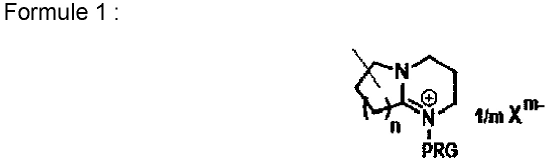 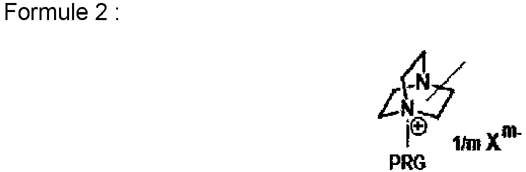 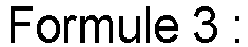 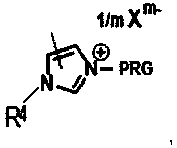 soit 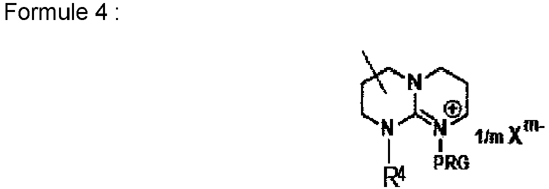
où : n est 1, 2 ou 3 ;
R⁴ représente l'hydrogène, un alkyle en C₁ à C₆, un phényle ou un substituant polymère représenté par la formule générale -L˙Y dans laquelle L est une liaison covalente ou un groupe de jonction organique et Y˙ est un radical polymère choisi dans le groupe constitué de polyéthers, polyesters, polycarbonates et leurs copolymères ; et,
X^{m-} est un contre-ion de la charge anionique m choisi parmi les halogénures et anions non coordinants comprenant un élément choisi parmi le bore, le phosphore ou le silicium.

7. Composé selon la revendication 5 ou la revendication 6,
où ledit radical polymère r-valent Y est un polyéther choisi dans le groupe constitué des oxydes de polyalkylène et de leurs copolymères.

8. Composé selon la revendication 7, où ledit radical polymère r-valent Y est soit un homopolymère linéaire d'oxyde d'éthylène ou d'oxyde de propylène, soit un copolymère linéaire d'oxyde d'éthylène, d'oxyde de propylène et, éventuellement, d'oxyde de butylène.

9. Composé selon l'une quelconque des revendications 5 à 8, où ledit radical polymère r-valent Y est **caractérisé par** un poids moléculaire moyen en poids de 100 à 100 000, de préférence de 400 à 7 500 g/mol.

10. Utilisation du composé tel que défini dans l'une quelconque des revendications 1 à 9 en tant que catalyseur latent dans une composition durcissable, dont le durcissement peut être catalysé ou co-catalysé par des amines tertiaires.

11. Utilisation selon la revendication 10, **caractérisée en ce que** ledit composé est exposé au rayonnement d'une lumière ultraviolette présentant :
une longueur d'onde de 150 à 600 nm, de préférence de 200 à 450 nm ; et/ou
une énergie de 5 à 500 mJ/cm², de préférence de 50 à 400 mJ/cm².

12. Composition de revêtement, adhésive ou d'étanchéité à base de polyuréthane comprenant :
a) un polyisocyanate ;
b) un polyol ; et,
c) un catalyseur latent comprenant au moins un composé tel que défini dans l'une quelconque des revendications 1 à 9.

13. Composition à base de résine époxy durcissable pour une composition de revêtement, adhésive ou d'étanchéité ou pour une matrice de matériaux composites, ladite composition à base de résine époxy comprenant :
a) une résine époxy ;
b) un catalyseur latent comprenant au moins un composé tel que défini dans l'une quelconque des revendications 1 à 9 ; et, éventuellement
c) un agent durcisseur pour ladite résine époxy.

14. Composition selon la revendication 12 ou 13, comprenant en outre un catalyseur en une quantité comprise entre 0,01 et 10 % en poids, de préférence entre 0,01 et 5 % en poids, par rapport au poids total de la composition.
